# EUROPÄISCHE PATENTANMELDUNG

(11) **EP 1 591 142 A1**
(43) Veröffentlichungstag der Anmeldung: **02.11.2005**
(21) Anmeldenummer: 05008989.5
(22) Anmeldetag: 25.04.2005
(51) Int. Cl.: A61N 1/40, A61N 2/02, A61K 41/00

(54) **Vorrichtung zur Wiedergabe und Verfahren zur Aufzeichnung und Wiedergabe von homöopathischen Informationen**

(30) Priorität: 26.04.2004 AT 31104 U
(71) Anmelder: Quintsysteme für holopathische Medizin Ges.m.b.H., A-3107 St. Pölten (AT)
(72) Erfinder: Dillinger, Klaus, Dipl. Ing., 3100 St. Pölten (AT); Steiner, Christian, Dr., 9073 Viktring (AT)
(74) Vertreter: Puchberger, Peter

(57) **Zusammenfassung**

Bei einem Verfahren zur Aufzeichnung von homöopathischen Informationen in Form von elektromagnetischen Schwingungen durch Digitalisierung analoger elektromagnetischer Spektren und Abspeichern der digitalisierten Spektren, wird erfindungsgemäß entweder zusätzlich zu den Spektren eine Sequenz für die Wiedergabe dieser bzw. ausgewählter Spektren gespeichert, oder es werden die Spektren in einer bestimmten Sequenz gespeichert, wobei die Sequenz eine bestimmte Aufeinanderfolge der Spektren, sowie Länge und Anzahl von Wiederholungen der einzelnen Spektren für die Wiedergabe als analoges Signal vorgibt. Mit einer erfindungsgemäßen Vorrichtung können nach einem erfindungsgemäßen Verfahren die homöopathischen Informationen dann in Form der Sequenz abgegeben werden um eine bessere Wirkung der homöopathischen Information zu erzielen.

## Beschreibung

Die Erfindung betrifft eine Vorrichtung zur Wiedergabe von homöopathischen Informationen in Form von elektromagnetischen Schwingungen basierend auf substanz- und/oder körperspezifischen Spektren, insbesondere zu therapeutischen Zwecken, mit einem Speicher, einem D/A-Konverter und einem Gerät zur Abgabe von elektromagnetischen Schwingungen, ein Verfahren zur Aufzeichnung von homöopathischen Informationen in Form von elektromagnetischen Schwingungen durch Digitalisierung analoger elektromagnetischer Spektren und Abspeichern der digitalisierten Spektren und ein Verfahren zur Wiedergabe von homöopathischen Informationen in Form von elektromagnetischen Schwingungen, wobei mehrere ausgewählte homöopathische Spektren in ein analoges Signal gewandelt und verstärkt werden und dann durch ein Gerät zur Abgabe von elektromagnetischen Schwingungen abgegeben werden.

Aus der Literatur, z.B. den österreichischen Gebrauchsmustern AT130U, AT1358U und AT4221U ist es bekannt, dass homöopathische Informationen in Form von elektromagnetischen Schwingungen aufgezeichnet, digital gespeichert und zu therapeutischen Zwecken über einen D/A-Konverter und ein Abgabegerät wiedergegeben werden können. Das kann z.B. ein herkömmliches Musikwiedergabegerät sein. Bisher wurde zu jedem Spektrum eine Dosierinformation, d.h. eine Information über die Dauer, die Zahl und den zeitlichen Abstand der Wiederholungen der Abgabe durch das Abgabegerät abgespeichert. In den seltensten Fällen ist jedoch die Anwendung eines einzigen Spektrums zufriedenstellend und es wurde bereits vorgeschlagen je nach Indikation(en) oder Krankheitssymptomen Spektrensummen und dazugehörige Dosierinformationen abzuspeichern.

Um die Wirkung der homöopathischen Information zu verbessern wird für das eingangs genannte Verfahren zur Wiedergabe erfindungsgemäß vorgeschlagen, dass die Spektren in Form einer Sequenz mit einer bestimmten Aufeinanderfolge der Spektren, Länge und Anzahl von Wiederholungen der einzelnen Spektren für die Wiedergabe in das analoge Signal gewandelt werden, wobei insbesondere die Sequenz mehrmals hintereinander abgearbeitet wird, bis ein vorgegebenes Zeitlimit oder Limit in der Anzahl der Wiederholungen der Sequenz erreicht ist. Durch das Abarbeiten der Sequenz werden die Spektren aufeinander aufbauend eingesetzt und so eine bessere Wirkung erzielt. Die Sequenz kann für sich oder gemeinsam mit der Information über das mehrfache Abarbeiten der Sequenz den Inhalt einer Therapiesitzung oder den Inhalt' mehrerer identer oder verschiedener aufeinanderfolgender Therapiesitzungen enthalten, die z.B. durch ein akustisches Signal von einander getrennt sind.

Beim erfindungsgemäßen eingangsgenannten Verfahren zur Aufzeichnung wird entweder zusätzlich zu den Spektren eine Sequenz für die Wiedergabe dieser bzw. ausgewählter Spektren gespeichert, oder es werden die Spektren in einer bestimmten Sequenz gespeichert, wobei die Sequenz eine bestimmte Aufeinanderfolge der Spektren, sowie Länge und Anzahl von Wiederholungen der einzelnen Spektren für die Wiedergabe als analoges Signal vorgibt. Zusätzlich können Informationen abgespeichert werden um die Sequenz mehrmals hintereinander abzuarbeiten, bis ein vorgegebenes Zeitlimit oder Limit in der Anzahl der Wiederholungen der Sequenz erreicht ist.

Eine entsprechende Vorrichtung zur Wiedergabe von homöopathischen Informationen der eingangsgenannten Art, weist in ihrem Speicher entweder zusätzlich zu den Spektren Informationen für die Wiedergabe ausgewählter Spektren in Form einer Sequenz oder eine Sequenz bestehend aus ausgewählten Spektren auf, wobei die Sequenz eine bestimmte Aufeinanderfolge der ausgewählten Spektren, sowie Länge und Anzahl von Wiederholungen der einzelnen ausgewählten Spektren bei der Wiedergabe als analoges Signal vorgibt.

Mit einer solchen Vorrichtung ist die Abgabe von homöopathischen Informationen einfach und sicher. Es wird keine Vorbereitungszeit vor der Anwendung benötigt und eine falsche Dosierung ist ausgeschlossen. Die Anwendung durch den Endverbraucher, insbesondere Patienten, ist ohne den Fachmann möglich. Die auf die Indikation(en) oder die Krankheitssymptome gegebenenfalls auch individuell durch den Homöopathen auf den Patienten abgestimmte Sequenz sichert eine gute Wirkung.

## Patentansprüche

1. Vorrichtung zur Wiedergabe von homöopathischen Informationen in Form von elektromagnetischen Schwingungen basierend auf substanz- und/oder körperspezifischen Spektren, insbesondere zu therapeutischen Zwecken, mit einem Speicher, einem D/A-Konverter und einem Gerät zur Abgabe von elektromagnetischen Schwingungen, **dadurch gekennzeichnet, dass** im Speicher zusätzlich zu den Spektren Informationen über die Aufeinanderfolge ausgewählter Spektren, sowie Länge und Anzahl von Wiederholungen der einzelnen ausgewählten Spektren für die Wiedergabe der ausgewählten Spektren in Form einer Sequenz gespeichert sind.

2. Vorrichtung zur Wiedergabe von homöopathischen Informationen in Form von elektromagnetischen Schwingungen basierend auf substanz- und/oder körperspezifischen Spektren, insbesondere zu therapeutischen Zwecken, mit einem Speicher, einem D/A-Konverter und einem Gerät zur Abgabe von elektromagnetischen Schwingungen, **dadurch gekennzeichnet, dass** im Speicher ausgewählte Spektren in einer Sequenz mit einer bestimmten Aufeinanderfolge der Spektren, sowie Länge und Anzahl von Wiederholungen der einzelnen Spektren für die Wiedergabe gespeichert sind.

3. Vorrichtung nach Anspruch 1 oder 2, **dadurch gekennzeichnet , dass** im Speicher Informationen abgespeichert sind, um die Sequenz mehrmals hintereinander abzuarbeiten, bis ein vorgegebenes Zeitlimit oder Limit in der Anzahl der Wiederholungen der Sequenz erreicht ist.

4. Verfahren zur Aufzeichnung von homöopathischen Informationen in Form von elektromagnetischen Schwingungen durch Digitalisierung analoger elektromagnetischer Spektren und Abspeichern der digitalisierten Spektren, **dadurch gekennzeichnet, dass** zusätzlich zu den Spektren Informationen über die Aufeinanderfolge ausgewählter Spektren, sowie die Länge und die Anzahl der Wiederholungen der einzelnen ausgewählten Spektren für die Wiedergabe in einer Sequenz gespeichert werden.

5. Verfahren zur Aufzeichnung von homöopathischen Informationen in Form von elektromagnetischen Schwingungen durch Digitalisierung analoger elektromagnetischer Spektren und Abspeichern der digitalisierten Spektren, **dadurch gekennzeichnet, dass** das Abspeichern in einer Sequenz mit einer bestimmten Aufeinanderfolge ausgewählter Spektren, sowie Länge und Anzahl von Wiederholungen der einzelnen ausgewählten Spektren erfolgt.

6. Verfahren nach Anspruch 4 oder 5, **dadurch gekennzeichnet, dass** im Speicher Informationen abgespeichert werden, um die Sequenz mehrmals hintereinander abzuarbeiten, bis ein vorgegebenes Zeitlimit oder Limit in der Anzahl der Wiederholungen der Sequenz erreicht ist.

7. Verfahren zur Wiedergabe von homöopathischen Informationen in Form von elektromagnetischen Schwingungen, wobei mehrere ausgewählte homöopathische Spektren in ein analoges Signal gewandelt und verstärkt werden und dann durch ein Gerät zur Abgabe von elektromagnetischen Schwingungen abgegeben werden, **dadurch gekennzeichnet, dass** die Spektren in Form einer Sequenz mit einer bestimmten Aufeinanderfolge der Spektren, Länge und Anzahl von Wiederholungen der einzelnen Spektren für die Wiedergabe in das analoge Signal gewandelt werden.

8. Verfahren nach Anspruch 7, **dadurch gekennzeichnet, dass** die Sequenz mehrmals hintereinander abgearbeitet wird, bis ein vorgegebenes Zeitlimit oder Limit in der Anzahl der Wiederholungen der Sequenz erreicht ist.
